# EUROPEAN PATENT SPECIFICATION

(11) **EP 0 463 718 B1**
(45) Date of publication and mention of the grant of the patent: **01.03.1995**
(21) Application number: 91303600.0
(22) Date of filing: 22.04.1991
(51) Int. Cl.: A61M 25/02

(54) **Catheter retainer**
Haltevorrichtung für einen Katheter
Dispositif de fixation d'un cathéter

(30) Priority: 02.05.1990 GB 9009840
(43) Date of publication of application: 02.01.1992
(73) Proprietor: E.R. SQUIBB & SONS, INC., Princeton New Jersey 08543-4000 (US)
(72) Inventor: Hollands, Keith G. M., Sompting, Sussex (GB)
(74) Representative: Cook, Anthony John

(56) References cited:
- DE-A- 2 362 947
- GB-A- 2 191 407
- GB-A- 2 199 499
- US-A- 4 392 854
- US-A- 4 579 120

## Description

This invention relates to a catheter retainer.

In U.S. Patent No.(US-A)4,579,120 (MacGregor) there is disclosed a device and method for anchoring and relieving the strain on a percutaneous lead. Such a lead may be used, for example, in a heart "pacemaker" system or as a neural stimulator. In the MacGregor arrangement, a thread is used to tie the lead to a boss which is located on a flange. The flange carries adhesive whereby it can be attached to the body of the wearer.

Catheter retainers are well known. For example, one form of catheter retainer is shown in European Patent No. 37198, and another example is shown in British Patent Application No. 2 199 499. Another method of retaining a catheter on the body of a patient is shown in British published patent application number 2 211 417. Many other designs are known. However, none of the catheter retainers presently commercially available are entirely satisfactory. Surgeons therefore still quite frequently stitch a catheter to the skin of the wearer. This is frequently done when it is desired to drain a wound and yet hold the wound largely closed. This procedure of course involves a risk of infection and may be painful for the patient.

Prior art catheter retainers are unsatisfactory for a number of reasons. Firstly the retainer may be size dependent. In other words, for catheters of different diameter, a different catheter retainer device may be required. Another disadvantage is that known catheter retainers are often hard and rigid, and therefore uncomfortable to wear. Wearing them may promote lesions or maceration of the skin and so give rise to problems other than that for which the patient is in hospital. Another disadvantage of known catheter retainers is that they are hard to clean. Also, when used with catheters which are of a silicone rubber, a material commonly used for catheters, the catheter tubes are hard to lock in position relative to the retainer because of the slipperyness of the tube. As a result, patients can inadvertently pull catheters out. Yet another disadvantage of known catheter retainers is that the clamping pressure which most of them need to apply tends to collapse the catheter or the drainage tube. In consequence, the efficency of drainage is adversely affected.

It would be desirable if there existed a catheter retainer which overcomes or at least greatly mitigates these disadvantages.

According to the invention, there is provided a catheter retainer comprising a pad of medical grade adhesive capable of attachment to the body of a person who has to use a catheter, in which the pad carries a member which cooperates with a cotton or synthetic fibre thread, or a thread of like material, the thread being of such a length that it can be wound around the catheter at least once and preferably several times and also wound around the member, and in which the member has a pair of upstanding ears each of which is provided with at least one slot for reception of the thread.

It has been found that this arrangement gives a very simple yet effective catheter retainer.

According to a preferred embodiment of the invention, the thread is attached at one of its ends to one of the ears, the other ear having a slot in which the free end of the thread can be engaged after one or more turns around the catheter have been taken.

The invention as defined above has the great advantage of simplicity. The catheter retainer is easy to fix to the patient, and the winding of the thread around the catheter can be done with one hand if needed. Moreover, one catheter retainer can take two or more catheters. This is important and advantageous when it is necessary to have one catheter supplying liquid to the interior of the body and the other catheter dealing with drainage. A catheter retainer of this kind has an automatic safety feature in that if the catheter is inadvertently pulled, the thread tends to tighten and hence grip the catheter more tightly. The catheter is easily released by unwinding the thread.

In a modification of the invention, multiple tubing having parallel liquid paths can also be securely attached to the body of a patient.

Either sufficient turns may be wound not to need anchoring of the free end or the thread after encircling the catheter may be anchored on another part of the catheter retainer device.

The invention will be better understood from the following illustrative description of one example, given with reference to the accompanying drawings, in which
Figure 1 is a perspective view of one example of catheter retainer according to the invention; and
Figure 2 is a second example.

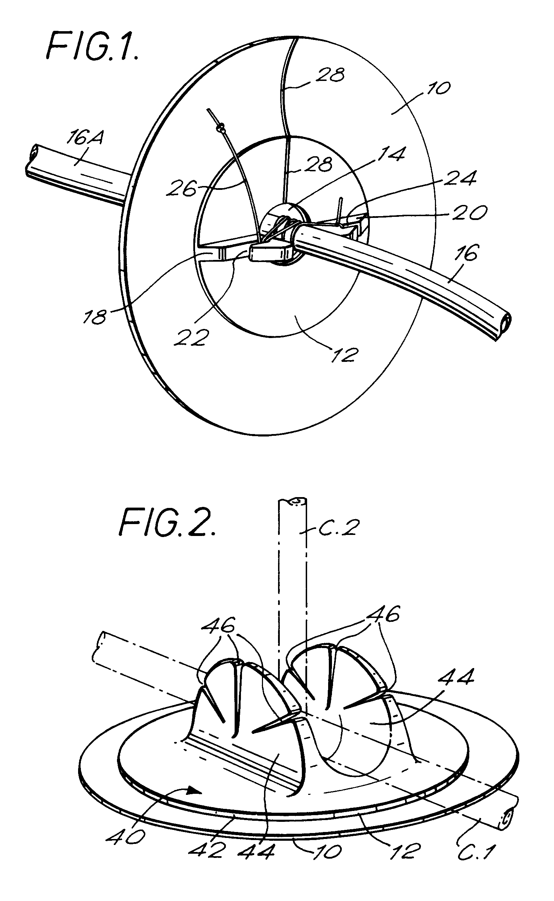

The catheter retainer illustrated in Figure 1 of the drawings comprises a pad 10 of medical grade adhesive material, which as seen is a circular pad and which has a backing 12 of a synthetic plastics film. As seen, the pad 10 is circular and the film is concentric therewith but has a smaller diameter. The pad 10 may for example be of the medical grade adhesive known by the Trade Mark "STOMAHESIVE", available from ConvaTec Limited, of Hounslow, Middlesex, England. A central circular hole 14 extends through both pad and film. It is sized slightly larger than the outside diameter of a catheter 16 which is to be passed through it. In the drawing, the portion 16a of the catheter is that within the body of the wearer. The hole 14 could be of any shape.

Secured to the plastics film 12 are two members 18 and 20, each having respective slots 22, 24. A thread 26, for example about 100 mm (10 cms) in length, is attached to one of the members 20 and its free end, is, in use, wound around the catheter 16 a number of times and then slipped into the slot 22. The slot 22 may be tapered so that it becomes narrower towards its inner end. The thread is gripped firmly by this kind of slot.

When it is desired to release the catheter, the free end of the thread 26 is removed from the slot 22 and unwrapped, so releasing the catheter which can then be withdrawn, while the pad 10 remains on the body of the wearer.

Of course by having a larger central hole, more than one catheter can be similarly accommodated.

Other variations are possible without departing from the invention. For example, the members 18 and 20 need not have the specific shape or form shown. So long as one anchor member is attached to the medical grade adhesive, serving for the attachment point for one end of the thread, then a catheter can be adequately retained by winding multiple turns of the thread around the catheter.

It is highly desirable, as shown, that the hole 14 should be connected by a cut or slit 28 to the exterior edge of the pad 10. In this way, the catheter can be put in place by passing it radially inwardly through this slot 28 rather than threading it through the hole 14 in a direction transverse to the plane of the pad 10.

The catheter retainer illustrated in Figure 2 also has a pad 10 of medical grade adhesive material which has a backing 12 of plastics film. Attached to the film is a thread attachment member 40 which may be of moulded plastics material. The member 40 has a disc or flange 42 which is attached (e.g. by adhesive) to the film 12. Two ears 44 are upstanding from the flange 42. Each of the ears is provided with three slots 46. These slots enable thread to be looped around in any desired manner so as to securely retain a catheter. Two possible positions for the catheter are shown at C.1 and C.2 The thread is not shown, for clarity, in Figure 2.

## Claims

1. A catheter retainer comprising a pad (10) of medical grade adhesive capable of attachment to the body of a person who has to use a catheter (16), in which the pad carries a member (18,20; or 44) which cooperates with a cotton or synthetic fibre thread (26), or a thread of like material, the thread being of such a length that it can be wound around the catheter at least once and preferably several times and also wound around the member, and in which the member has a pair of upstanding ears each of which is provided with at least one slot (22,24; or 46) for reception of the thread.

2. A retainer according to claim 1 in which the thread is attached at one of its ends to one of the ears, the other ear having a slot (22 or 24) in which the free end of the thread can be engaged after one or more turns around the catheter have been taken.

3. A retainer according to claim 1 in which the thread has one end attached to the member (18,20; or 44).

## Patentansprüche

1. Haltevorrichtung für einen Katheter mit einer Auflage (10) aus einem für medizinische Zwecke einsetzbaren Klebstoff, die zum Anlegen an den Körper einer auf einen Katheter (16) angewiesenen Person geeignet ist, wobei die Auflage ein Element (18, 20; bzw. 44) trägt, das mit einem Baumwoll- oder Kunststoffaserfaden (26) oder einem Faden ähnlichen Materials zusammenwirkt, und der Faden so lang ist, daß er um den Katheter mindestens einmal, vorzugsweise mehrmals, und auch um das Element geschlungen werden kann, und das Element ein Paar aufrecht stehender Laschen aufweist, von denen jede mit mindestens einem Schlitz (22, 24, bzw. 46) zur Aufnahme des Fadens versehen ist.

2. Haltevorrichtung nach Anspruch 1, bei der ein Ende des Fadens an der einen Lasche befestigt ist und die andere Lasche einen Schlitz (22 bzw. 24) aufweist, in welchen das freie Ende des Fadens eingeklemmt werden kann, nachdem der Faden ein- oder mehrmals um den Katheter geschlungen wurde.

3. Haltevorrichtung nach Anspruch 1, wobei ein Ende des Fadens an dem Element (18, 20; bzw. 44) befestigt ist.

## Revendications

1. Dispositif de maintien de cathéter comprenant un tampon (10) d'adhésif de qualité médicale pouvant être fixé au corps d'une personne qui doit utiliser un cathéter (16), dans lequel le tampon porte une pièce (18, 20; ou 44) qui coopère avec un fil de coton ou de fibre synthétique (26), ou avec un fil de matière similaire, le fil ayant une longueur telle qu'il peut être enroulé autour du cathéter au moins une fois et de préférence plusieurs fois et qu'il peut aussi être enroulé autour de la pièce, et dans lequel la pièce comporte deux oreilles saillantes dont chacune est munie d'au moins une fente (22, 24; ou 46) de réception du fil.

2. Dispositif de maintien selon la revendication 1, dans lequel le fil est fixé par l'une de ses extrémités à l'une des oreilles, l'autre oreille comportant une fente (22 ou 24) dans laquelle on peut engager l'extrémité libre du fil après lui avoir fait faire un ou plusieurs tours autour du cathéter.

3. Dispositif de maintien selon la revendication 1, dans lequel le fil a l'une de ses extrémités fixée à la pièce (18, 20; ou 44).
